# EUROPÄISCHE PATENTSCHRIFT

(11) **EP 3 294 256 B1**
(45) Veröffentlichungstag und Bekanntmachung des Hinweises auf die Patenterteilung: **06.11.2019**
(21) Anmeldenummer: 16722192.8
(22) Anmeldetag: 11.05.2016
(51) Int. Cl.: A61J 1/20, A61M 39/04, A61M 39/20, A61M 39/26, A61J 1/14, A61M 39/10

(54) **BEHÄLTNIS FÜR EINE MEDIZINISCHE FLÜSSIGKEIT**
CONTAINER FOR A MEDICAL LIQUID
RÉCIPIENT POUR UN LIQUIDE MÉDICAL

(30) Priorität: 13.05.2015 EP 15167619
(43) Veröffentlichungstag der Anmeldung: 21.03.2018
(73) Patentinhaber: Fresenius Kabi Deutschland GmbH, 61352 Bad Homburg (DE)
(72) Erfinder: BRANDENBURGER, Torsten, 61203 Reichelsheim (DE); RAHIMY, Ismael, 61169 Friedberg (DE); FRENSCH, Christian, 65719 Hofheim (DE)
(74) Vertreter: Fresenius Kabi Deutschland GmbH
(86) Internationale Anmeldenummer: PCT/EP2016/060539
(87) Internationale Veröffentlichungsnummer: WO 2016/180869

(56) Entgegenhaltungen:
- WO-A1-2013/017698
- WO-A1-2015/007703

## Beschreibung

Die Erfindung betrifft ein Behältnis für eine medizinische Flüssigkeit nach dem Oberbegriff des Anspruch 1.

Ein derartiges Behältnis umfasst ein oder mehrere Anschlussstücke, über die eine medizinische Flüssigkeit in das Behältnis hinein oder aus dem Behältnis heraus gefördert werden kann. An zumindest eines dieser Anschlussstücke kann ein Ansetzteil angesetzt werden, das mit einer Fördereinrichtung, z.B. einer Spritze, zum Fördern einer medizinischen Flüssigkeit in das Behältnis hinein oder aus dem Behältnis heraus verbunden werden kann. Ein Dichtelement dichtet einen Übergang zwischen dem Anschlussstück und dem Ansetzteil ab, wobei das Dichtelement im Allgemeinen eine Schlitzöffnung aufweist, die bei nicht an das Ansetzteil angesetzter Fördereinrichtung gegen einen Flüssigkeitsdurchtritt geschlossen ist und durch Ansetzen der Fördereinrichtung an das Ansetzteil geöffnet werden kann derart, dass eine medizinische Flüssigkeit durch die Schlitzöffnung hindurch gefördert werden kann.

Ein solches Behältnis kann beispielsweise als flexibler Beutel oder auch als Ampulle oder sonstiges Flaschenbehältnis ausgestaltet sein.

Bei einem aus der WO 2005/037362 A1 bekannten Konnektor für eine eine medizinische Flüssigkeit enthaltende Verpackung ist in eine kanalförmige Ausnehmung eines Anschlussteils eine selbstabdichtende Membran eingesetzt. Die kanalförmige Ausnehmung ist durch ein Abbrechteil verschlossen, das zum Freigeben der kanalförmigen Ausnehmung von dem Anschlussteil abgebrochen werden kann, so dass eine Spritze mit einem Anschlussstutzen an das Anschlussteil angesetzt werden kann. Durch Ansetzen der Spritze an das Anschlussteil kann die Membran geöffnet werden, so dass eine Flüssigkeit in ein Behältnis hinein oder aus dem Behältnis heraus gefördert werden kann. In der WO 2015/007703 A1 ist eine entsprechende Ampulle für eine medizinische Flüssigkeit beschrieben.

Bei einem aus der WO 2010/034470 A1 bekannten Konnektor ist an einer Seite einer Membran, die einer an die Membran anzusetzenden Spritze abgewandt ist, ein hohlförmiger Körper mit einer Spitze angeordnet. Bei Ansetzen der Spritze an den Konnektor wird die Membran durch die Spritze auf die Spitze des hohlförmigen Körpers gedrückt, so dass die Spritze mit dem hohlförmigen Körper in Eingriff gelangt und somit ein Durchfluss durch die Membran ermöglicht wird.

In der WO 2013/017698 A1 ist ein nadelfreier Konnektor beschrieben. Dieser beinhaltet einen Ventilkörper mit einer ersten und einer zweiten Öffnung. Eine hohle elastische Membran ist in dem Ventilkörper angeordnet. Die Membran besitzt ein erstes und ein zweites Ende. Die Membran weist eine Seitenwand auf, welche das erste Ende mit dem zweiten Ende verbindet. Die Seitenwand der Membran liegt an einer inneren Oberfläche des Ventilkörpers an.

Aufgabe der vorliegenden Erfindung ist es, ein Behältnis mit einem Anschlussstück und einem daran ansetzbaren Ansetzteil zur Verfügung zu stellen, bei dem ein großer Durchfluss, insbesondere mit geringem Kraftaufwand, zum Fördern einer medizinischen Flüssigkeit in das Behältnis hinein oder aus dem Behältnis heraus erhalten werden kann.

Diese Aufgabe wird durch einen Gegenstand mit den Merkmalen des Anspruchs 1 gelöst.

Anspruch 1 beschreibt ein Behältnis für eine medizinische Flüssigkeit mit einem Anschlussstück, über das eine medizinische Flüssigkeit in das Behältnis hinein oder aus dem Behältnis heraus förderbar ist, einem an das Anschlussstück ansetzbaren Ansetzteil, das mit einer Förderreinrichtung zum Fördern einer medizinischen Flüssigkeit in das Behältnis hinein oder aus dem Behältnis heraus verbindbar ist, und einem Dichtelement zum Abdichten eines Übergangs zwischen dem Anschlussstück und dem Ansetzteil, wobei das Dichtelement eine Schlitzöffnung aufweist, die bei nicht an das Ansetzteil angesetzter Fördereinrichtung gegen einen Flüssigkeitsdurchtritt geschlossen und durch Ansetzen der Fördereinrichtung an das Ansetzteil zu öffnen ist derart, dass eine medizinische Flüssigkeit durch die Schlitzöffnung hindurch förderbar ist. Das Ansetzteil weist einen Verbindungsabschnitt auf mit einer Öffnung, in die das Anschlussstück zum Verbinden mit dem Ansetzteil entlang einer Einsetzrichtung (E) einsetzbar ist. Die Öffnung weist einen ersten Aufnahmeabschnitt und einen entlang der Einsetzrichtung (E) axial daran anschließenden, zweiten Aufnahmeabschnitt auf, wobei bei an das Anschlussstück angesetztem Ansetzteil der erste Aufnahmeabschnitt einen Kopf des Anschlussstücks und der zweite Aufnahmeabschnitt einen Abschnitt des Dichtelements aufnimmt.

Gemäß der Erfindung weist der erste Aufnahmeabschnitt einen ersten Durchmesser D1 und der zweite Aufnahmeabschnitt einen zweiten Durchmesser D2 auf, wobei der erste Durchmesser D1 kleiner ist als der zweite Durchmesser D2 und das Dichtelement bei nicht an das Ansetzteil angesetzter Fördereinrichtung mit radialem Spiel in oder an dem zweiten Aufnahmeabschnitt aufgenommen ist. Dies ermöglicht, dass das Dichtelement mit Spiel in dem zweiten Aufnahmeabschnitt einliegen und dabei dennoch zuverlässig klemmend zwischen dem Anschlussteil und dem Ansetzteil gehalten werden kann. Weil das Dichtelement mit Spiel in dem zweiten Aufnahmeabschnitt einliegt, kann es sich bei Ansetzen einer Fördereinrichtung, insbesondere einer Spritze, radial verformen und somit durch die Fördereinrichtung beiseite gedrängt werden, so dass die in dem Dichtelement vorgesehene Schlitzöffnung in zuverlässiger Weise unter Verformung des Dichtelements geöffnet und, vorzugsweise vollständig, freigegeben werden kann.

In einer Ausführungsform ist bei dem Behältnis vorgesehen, dass das Dichtelement aus einem thermoplastischen Elastomer gefertigt ist. Insbesondere weist das thermoplastische Elastomer (TPE) hierbei eine Shore-Härte A zwischen 25 und 70, vorzugsweise zwischen 50 und 60 auf. Vorzugsweise ist oder umfasst das thermoplastische Elastomer ein TPE-Material vom Typ Styrol-TPE.

Bei der genannten Shore-Härte A handelt es sich um einen Werkstoffkennwert für thermoplastische Elastomere, der in der Norm DIN ISO 7619-1 festgelegt ist. Bei einer Shore-Härteprüfung wird ein federbelasteter Stift aus gehärtetem Stahl auf einen zu prüfenden Werkstoff aufgesetzt und die Eindringtiefe in den Werkstoff geprüft. Eine hohe Shore-Härte bedeutet, dass der Werkstoff eine große Härte aufweist. Generell wird die Shore-Härte auf einer Skala von bis 0 bis 100 gemessen. Die Spitze des Stahl-Stifts drückt bei Shore A bzw. dringt bei Shore D in das Material ein. Die Eindruck-/Eindringtiefe wird auf einer Skala von 0 -100 gemessen. Der Stahl-Stift hat entweder die Geometrie eines Kegelstumpfes (Shore A) oder einer Nadelspitze (Shore D).

Bei herkömmlichen Behältnissen kann das Problem bestehen, dass sich das Dichtelement, beispielsweise in Form einer Membran, beim Ansetzen einer Fördereinrichtung, insbesondere einer Spritze, nicht vollständig öffnet. Dies kann ein nochmaliges Ansetzen der Fördereinrichtung erforderlich machen oder aber den Durchfluss durch das Anschlussstück beeinträchtigen. Im Stand der Technik ist dazu ein zusätzlicher hohlförmiger Körper mit einer Spitze vorgesehen, der das Öffnen der Membran unterstützen soll. Bei dem erfindungsgemäßen Konnektor ist jedoch kein zusätzlicher hohlförmiger Körper vorgesehen. Während herkömmliche Dichtelemente aus einem vergleichsweise weichen Material gefertigt sein können, ist das Dichtelement in einer Ausführungsform aus einem thermoplastischen Elastomer, vorzugsweise mit einer vergleichsweise großen Shore-Härte, gefertigt. In einer alternativen Ausführungsform ist das Dichtelement aus Polyisopren gefertigt.

In einer Ausführungsform ist das Dichtelement derart beschaffen, dass bei Ansetzen einer Fördereinrichtung, insbesondere einer Spritze, an das Ansetzteil das Dichtelement zum Öffnen der Schlitzöffnung hinreichend verformt werden kann und eine hinreichend große Durchflussöffnung freigibt. Die Fördereinrichtung erstreckt sich dann, wenn sie an das Ansetzstück angesetzt ist, vorzugsweise mit einem Anschlussstutzen, durch die Schlitzöffnung des Dichtelements hindurch, so dass ein Durchfluss zwischen der Fördereinrichtung und dem Behältnis ungehindert durch das Dichtelement erfolgen kann. In einer Ausführungsform sind mindestens 80 %, vorzugsweise mindestens 90 %, des Öffnungsquerschnitts in der Fördereinrichtung, durch welche der Flüssigkeitstransport erfolgt, freigegeben und somit nicht durch die Dichtung verdeckt. Vorzugsweise ist der gesamte Querschnitt der Öffnung in der Fördereinrichtung, durch welche der Flüssigkeitstransport erfolgt, freigegeben.

Beispielsweise kann eine größere Härte des Dichtelements einen vorteilhaften Durchfluss ermöglichen, weil das Dichtelement größerer Härte - insbesondere im Vergleich zu einem weicheren Dichtelement - vorteilhaft zum Öffnen der Schlitzöffnung bei Ansetzen der Fördereinrichtung, insbesondere eines Anschlussstutzens einer Spritze, verformt werden kann. Insbesondere kann das Dichtelement radial beiseite gedrängt werden, ohne dass eine übermäßige Verformung in axialer Richtung auftritt und insbesondere das Dichtelement bei Ansetzen der Fördereinrichtung in Axialrichtung sich nicht übermäßig dehnen kann.

Erfindungsgemäß weist das Ansetzteil einen Verbindungsabschnitt mit einer Öffnung auf, in die das Anschlussstück zum Verbinden mit dem Ansetzteil entlang einer Einsetzrichtung eingesetzt werden kann. Durch Einsetzen des Anschlussstücks in die Öffnung des Ansetzteils wird das Anschlussstück formschlüssig mit dem Ansetzteil verbunden, wobei das Dichtelement in klemmender Weise zwischen dem Ansetzteil und dem Anschlussstück zu liegen kommt und somit in dichtender Weise zwischen dem Ansetzteil und dem Anschlussstück gehalten ist.

Die Öffnung kann eine im Wesentlichen zylindrische Grundform aufweisen. In der Öffnung ist hierbei ein erster Aufnahmeabschnitt ausgebildet, an den - betrachtet entlang der Einsetzrichtung - axial ein zweiter Aufnahmeabschnitt anschließt. Ist das Ansetzteil an das Anschlussstück angesetzt, so nimmt der erste Aufnahmeabschnitt einen Kopf des Anschlussstücks, vorzugsweiseformschlüssig, auf, während in dem zweiten Aufnahmeabschnitt ein beispielsweise rotationssymmetrischer Körper des Dichtelements zu liegen kommt.

Hierbei ist der erste Aufnahmeabschnitt in seinem Durchmesser kleiner als der zweite Aufnahmeabschnitt. Dies ermöglicht, dass das Dichtelement mit Spiel in dem zweiten Aufnahmeabschnitt einliegen und dabei dennoch zuverlässig klemmend zwischen dem Anschlussteil und dem Ansetzteil gehalten werden kann. Weil das Dichtelement mit Spiel in dem zweiten Aufnahmeabschnitt einliegt, kann es sich bei Ansetzen einer Fördereinrichtung, insbesondere einer Spritze, radial verformen und somit durch die Fördereinrichtung beiseite gedrängt werden, so dass die in dem Dichtelement vorgesehene Schlitzöffnung in zuverlässiger Weise unter Verformung des Dichtelements geöffnet werden kann.

Über den ersten Aufnahmeabschnitt werden das Ansetzteil und das Anschlussstück formschlüssig miteinander verbunden. An dem ersten Aufnahmeabschnitt kann hierbei eine sich um die Einsetzrichtung herum erstreckende Innenverzahnung vorgesehen sein, die bei an das Anschlussstück angesetztem Ansetzteil formschlüssig mit einer zugeordneten Verzahnung an dem Kopf des Anschlussstücks in Eingriff gelangt, so dass das Ansetzteil und das Anschlussstück drehfest um die Einsetzrichtung zueinander festgelegt sind. Nach Ansetzen des Ansetzteils an das Anschlussstück können das Ansetzteil und das Anschlussstück somit nicht (mehr) zueinander verdreht werden, so dass das Ansetzteil an dem Anschlussstück festgelegt ist.

In einer Ausführungsform weisen sowohl der erste Aufnahmeabschnitt als auch der zweite Aufnahmeabschnitt eine Verzahnung, beispielsweise in Form einer umlaufenden Innenverzahnung, auf. Die Verzahnung des ersten Aufnahmeabschnitts dient der drehfesten Festlegung an dem Kopf des Anschlussstücks. Die Verzahnung des zweiten Aufnahmeabschnitts dient der verbesserten Entformung des Ansetzteils nach dem Kunststoffspritzgießen bei der Herstellung.

Das Dichtelement weist in einer vorteilhaften Ausgestaltung einen zylindrischen Körper, der bei an das Anschlussstück angesetztem Ansetzteil klemmend zwischen dem Anschlussstück und dem Ansetzteil gehalten wird, und einen an den Körper anschließenden Dichtkopf, in dem die Schlitzöffnung ausgebildet ist, auf. Der Körper kann hierbei einen größeren Durchmesser als der Dichtkopf (betrachtet radial zur Einsetzrichtung) aufweisen. Das Dichtelement ist vorzugsweise insgesamt rotationssymmetrisch mit einem zylindrischen Körper und einem daran anschließenden Dichtkopf ausgestaltet.

Vorzugsweise ist der Dichtkopf an einer von dem Körper abgewandten Seite eben ausgebildet. Dies ermöglicht, wenn eine Fördereinrichtung, insbesondere eine Spritze, an das Ansetzteil angesetzt wird, dass der Dichtkopf durch Einführen eines Anschlussstutzens der Fördereinrichtung in die Schlitzöffnung in vorteilhafter Weise beiseite gedrängt werden kann, so dass bei vollständig an das Ansetzteil angesetzter Fördereinrichtung der Anschlussstutzen den Dichtkopf des Dichtelements durchdringt und somit ein Durchfluss zwischen der Fördereinrichtung und dem Behältnis nicht durch das Dichtelement beeinträchtig ist.

Denkbar und möglich ist jedoch auch, dass der Dichtkopf an seiner von dem Körper abgewandten Seite konvex nach außen oder konkav nach innen gewölbt ist.

Der Körper des Dichtelements weist vorzugsweise eine, vorzugsweise zylindrische oder im Wesentlichen zylindrische, durch den Dichtkopf axial begrenzte Bohrung auf, die vorzugsweise mit konstantem Durchmesser über ihre gesamte Höhe (betrachtet entlang der Einsetzrichtung) ausgebildet ist oder sich hin zum Dichtkopf verjüngt. Dadurch, dass die, vorzugsweise zylindrische, Bohrung mit einem - im Vergleich zu einer Durchflussöffnung eines Anschlussstutzens einer Fördereinrichtung - großen Durchmesser ausgebildet ist, kann sichergestellt werden, dass das Dichtelement mit seinem Körper einen Durchfluss nicht behindert, wenn die Fördereinrichtung an das Ansetzteil angesetzt ist.

Um das Anbringen des Ansetzteils an dem Anschlussstück zu erleichtern, kann an dem Dichtelement ein radial von dem Körper vorstehendes Formschlusselement, beispielsweise in Form eines umlaufenden Kranzes, vorgesehen sein, das dazu dient, das Dichtelement an dem Ansetzteil zu halten, wenn es in die Öffnung des Ansetzteils eingesetzt ist. Das Anbringen des Ansetzteils an dem Anschlussstück kann somit zusammen mit dem in die Öffnung des Ansetzteils eingesetzten Dichtelement erfolgen, ohne dass besondere Vorsorge dafür getroffen werden müsste, das Dichtelement in Position zu dem Ansetzteil zu halten.

Das Ansetzteil weist, in einer vorteilhaften Ausgestaltung, einen Gewindeabschnitt mit mindestens einem Gewindegang zum Herstellen einer Gewindeverbindung mit einer an das Ansetzteil anzusetzenden Fördereinrichtung auf. Der Gewindeabschnitt kann beispielsweise einen Luer-Ansatz bereitstellen, mittels dessen das Abschlussteil nach Art eines sogenannten Luer-Locks mit einer Fördereinrichtung, beispielsweise einer Spritze, verbunden werden kann. Die Fördereinrichtung kann hierzu ein Anschlusselement in Form einer Überwurfmutter mit einem Innengewinde aufweisen, die mit dem Gewindeabschnitt in Gewindeeingriff gebracht werden kann, so dass über den Gewindeabschnitt die Fördereinrichtung lösbar mit dem Behältnis verbunden werden kann.

An den Gewindeabschnitt schließt vorzugsweise - in einem Ausgangszustand vor Ansetzen einer Fördereinrichtung an das Ansetzteil - ein Abbrechstück an, das in einem mit dem Gewindeabschnitt verbundenen Zustand eine Öffnung des Gewindeabschnitts verschließt und zum Freigeben der Öffnung von dem Gewindeabschnitt entfernt, insbesondere manuell abgebrochen werden kann. Über das Abbrechstück ist das Ansetzteil in seinem Ausgangszustand verschlossen, so dass der über das Anschlussstück bereitgestellte Zugang zum Behältnis sicher versiegelt ist. Zum Freigeben kann das Abbrechstück von dem Gewindeabschnitt entfernt, insbesondere abgebrochen werden, wobei hierzu zwischen dem Abbrechstück und dem Gewindeabschnitt eine definierte Sollbruchstelle vorgesehen sein kann, an der das Abbrechstück in definierter Weise von dem Gewindeabschnitt getrennt werden kann. Bei abgebrochenem Abbrechstück ist dann die Öffnung des Gewindeabschnitts freigegeben, so dass eine Fördereinrichtung an das Ansetzteil zum Hinein- oder Herausfördern einer Flüssigkeit angesetzt werden kann. Vorzugsweise ist der durch das Abbrechstück verschlossene Innenraum des Ansetzteils in dem Ausgangszustand, in dem das Abbrechstück noch nicht entfernt ist, bereits steril.

Im Bereich der Erfindung liegt auch ein Konnektor, insbesondere für eine Ausführungsform des vorstehend beschriebenen Behältnisses. Der Konnektor umfasst ein Anschlussstück, durch das eine medizinische Flüssigkeit förderbar ist, ein mit dem Anschlussstück, vorzugsweise über Ansetzen, verbundenes Ansetzteil, das mit einer Förderreinrichtung zum Fördern einer medizinischen Flüssigkeit durch das Anschlussstück verbindbar ist, und ein Dichtelement zum Abdichten eines Übergangs zwischen dem Anschlussstück und dem Ansetzteil. Das Dichtelement weist eine Schlitzöffnung aufweist, die bei nicht an das Ansetzteil angesetzter Fördereinrichtung gegen einen Flüssigkeitsdurchtritt geschlossen und durch Ansetzen der Fördereinrichtung an das Ansetzteil zu öffnen ist derart, dass eine medizinische Flüssigkeit durch die Schlitzöffnung hindurch förderbar ist. Das Ansetzteil weist einen Verbindungsabschnitt auf mit einer Öffnung, in die das Anschlussstück zum Verbinden mit dem Ansetzteil entlang einer Einsetzrichtung (E) einsetzbar ist. Die Öffnung weist einen ersten Aufnahmeabschnitt und einen entlang der Einsetzrichtung (E) axial daran anschließenden, zweiten Aufnahmeabschnitt auf. Bei an das Anschlussstück angesetztem Ansetzteil nimmt der erste Aufnahmeabschnitt einen Kopf des Anschlussstücks und der zweite Aufnahmeabschnitt einen Abschnitt des Dichtelements auf. Der Konnektor ist dadurch gekennzeichnet, dass der erste Aufnahmeabschnitt einen ersten Durchmesser D1 und der zweite Aufnahmeabschnitt einen zweiten Durchmesser D2 aufweist, wobei der erste Durchmesser D1 kleiner ist als der zweite Durchmesser D2 und das Dichtelement bei nicht an das Ansetzteil angesetzter Fördereinrichtung mit radialem Spiel in dem zweiten Aufnahmeabschnitt aufgenommen ist. In einer Ausgestaltung ist das Dichtelement aus einem thermoplastischen Elastomer gefertigt. Für mögliche weitere Ausführungsformen des Konnektors wird auf die vorstehende Beschreibung Bezug genommen.

Ein Dichtelement für eine Ausführungsform des vorstehend beschriebenen Behältnisses und/oder des vorstehend beschriebenen Konnektors ist dadurch gekennzeichnet, dass es einen Körper mit einem Außendurchmesser zwischen 6 mm und 10 mm und einen an den Körper anschließenden Dichtkopf mit einem Außendurchmesser zwischen 4 mm und 7 mm aufweist. In einer Ausgestaltung ist das Dichtelement aus einem thermoplastischen Elastomer gefertigt. Für mögliche weitere Ausführungsformen des Dichtelements wird auf die vorstehende Beschreibung Bezug genommen.

Bei einer Anordnung eines Behältnisses nach der vorangehend beschriebenen Art und einer Fördereinrichtung kann die Fördereinrichtung mit einem Anschlussstutzen an das mit dem Anschlussstück verbundene Ansetzteil angesetzt werden. Hierbei gelangt der Anschlussstutzen in Kontakt mit dem Dichtelement innerhalb des Ansetzteils und drängt das Dichtelement beiseite derart, dass bei angesetzter Fördereinrichtung der Anschlussstutzen die Schlitzöffnung des Dichtelements, vorzugsweise vollständig, durchdringt und somit ein Durchfluss zwischen der Fördereinrichtung und dem Behältnis nicht oder nicht wesentlich durch das Dichtelement beeinträchtigt ist.

Das Dichtelement kann beispielsweise einen Außendurchmesser zwischen 6 mm und 10 mm, vorzugsweise zwischen 7 mm und 9 mm, beispielsweise 8 mm, aufweisen, entsprechend dem Außendurchmesser des Körpers des Dichtelements.

Der Durchmesser des an den Körper anschließenden Dichtkopfs des Dichtelements kann demgegenüber zwischen 4 mm und 7 mm, beispielsweise zwischen 4,5 mm und 5,5 mm, insbesondere 5,1 mm, betragen.

Die Gesamthöhe des Dichtelements kann beispielsweise zwischen 4 mm und 7 mm, beispielsweise zwischen 4,5 mm und 6 mm, insbesondere 5,1 mm, betragen.

Die innere Bohrung des Dichtelements kann beispielsweise einen Durchmesser zwischen 3,5 mm und 5 mm, beispielsweise 4,2 mm, und eine axiale Höhe zwischen 2,5 mm und 5 mm, beispielsweise zwischen 3,5 mm und 4 mm, insbesondere 3,8 mm, aufweisen.

Das Ansetzteil kann an dem Verbindungsabschnitt beispielsweise einen Außendurchmesser zwischen 9 mm und 13 mm, beispielsweise zwischen 10 mm und 11 mm, insbesondere 10,2 mm, aufweisen.

Das Ansetzteil kann, insbesondere bei der Verwendung an einem Behältnis, eine Gesamthöhe (mit verbundenem Abbrechstück) zwischen 20 mm und 40 mm, beispielsweise zwischen 25 mm und 35 mm, insbesondere zwischen 28 mm und 30 mm, aufweisen.

Das Anschlussstück hingegen kann an seinem in den Verbindungsabschnitt des Ansetzteils einzusetzenden Kopf beispielsweise einen Durchmesser zwischen 7 mm und 12 mm, beispielsweise zwischen 8 und 10 mm, aufweisen.

Die Gesamthöhe des Anschlussstücks kann beispielsweise zwischen 30 mm und 50 mm, beispielsweise zwischen 35 mm und 45 mm, insbesondere zwischen 38 mm und 40 mm, betragen.

Der der Erfindung zugrunde liegende Gedanke soll nachfolgend anhand der in den Figuren dargestellten Ausführungsbeispiele näher erläutert werden. Es zeigen:
- Fig. 1: eine Ansicht eines Behältnisses in Form eines Beutels mit daran vorgesehenen Anschlussstücken, die durch Ansetzteile verschlossen sind;
- Fig. 2A: eine Seitenansicht eines Ansetzteils;
- Fig. 2B: eine Schnittansicht durch das Ansetzteil gemäß Fig. 2A;
- Fig. 3A: eine Schnittansicht durch ein an das Ansetzteil anzusetzendes Dichtelement;
- Fig. 3B: eine vergrößerte Ansicht der Schnittansicht aus Fig. 3A;
- Fig. 3C: eine Außenansicht des an das Ansetzteil anzusetzenden Dichtelements;
- Fig. 4: eine Seitenansicht eines Anschlussstücks, an das das Ansetzteil anzusetzen ist;
- Fig. 5: eine Schnittansicht durch das Ansetzteil und das Anschlussstück in einem aneinander angesetzten Zustand;
- Fig. 6: eine Schnittansicht durch ein anderes Ausführungsbeispiel eines Ansetzteils;
- Fig. 7: eine Ansicht einer Fördereinrichtung vor Ansetzen an einen Gewindeabschnitt eines Ansetzteils;
- Fig. 8: eine schematische Ansicht des Ansetzteils mit darin angeordnetem Dichtelement nach Ansetzen einer Fördereinrichtung in Form einer Spritze;
- Fig. 9: eine Ansicht eines weiteren Ausführungsbeispiels eines Behältnisses in Form eines Beutels mit daran angeordneten Anschlussstücken, die durch Ansetzteile verschlossen sind; und
- Fig. 10: eine Explosionsansicht eines Anschlussstücks mit einem daran anzusetzenden Ansetzteil in Form einer Blindkappe.

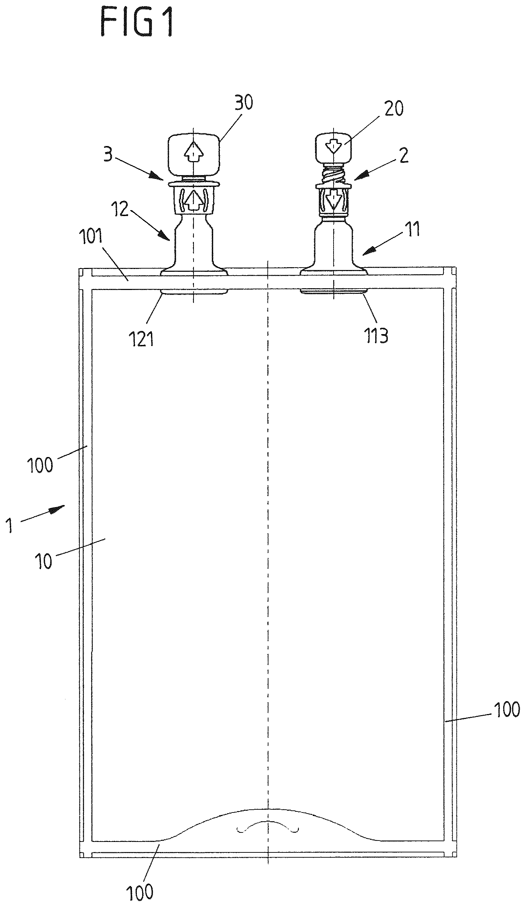

Fig. 1 zeigt eine schematische Ansicht eines Behältnisses 1 in Form eines flexiblen Beutels zum Aufbewahren einer medizinischen Flüssigkeit, beispielsweise eines Medikaments, einer Kochsalzlösung, einer Nährstofflösung zur enteralen oder parenteralen Ernährung oder dergleichen. Der Beutel 1 umfasst einen Beutelkörper 10, der zur Aufnahme der medizinischen Flüssigkeit ausgebildet ist und in an sich bekannter Weise ein Volumen, in den hinein eine medizinische Flüssigkeit eingefüllt oder aus dem heraus die medizinische Flüssigkeit entnommen werden kann, einschließt.

Bereits an dieser Stelle sei darauf hingewiesen, dass die vorliegende Erfindung nicht auf einen flexiblen Beutel beschränkt ist, sondern grundsätzlich auf ganz unterschiedliche Behältnisse 1, insbesondere auch Ampullen oder sonstige Flaschen zum Aufbewahren medizinischer Flüssigkeiten, angewendet werden kann.

Das Behältnis 1 umfasst zwei Anschlussstücke 11, 12, die zwei Zugänge in das Innere des Behältnisses 1 bereitstellen. Ein erstes Anschlussstück 11 ist hierbei insbesondere zum Zuführen einer medizinischen Flüssigkeit in das Behältnis 1 hinein vorgesehen, während ein zweites Anschlussstück 12 zum Entnehmen von Flüssigkeit aus dem Inneren des Behältnisses 1 dient.

Bei dem dargestellten Ausführungsbeispiel sind an beide Anschlussstücke 11, 12 Ansetzteile 2, 3 in Form von Kappen angesetzt. Über die Ansetzteile 2, 3 sind die Anschlussstücke 11, 12 nach außen hin flüssigkeitsdicht verschlossen, so dass keine Flüssigkeit aus dem Behältnis 1 heraus gelangen kann und das Behältnis 1 nach außen hin gegen das Eindringen von Schmutz oder dergleichen versiegelt ist.

Jedes Ansetzteil 2, 3 weist ein Abbrechstück 20, 30 als Originalitätsverschluss auf, das abgebrochen werden kann, um einen Zugang zu dem Behältnis 1 zu schaffen.

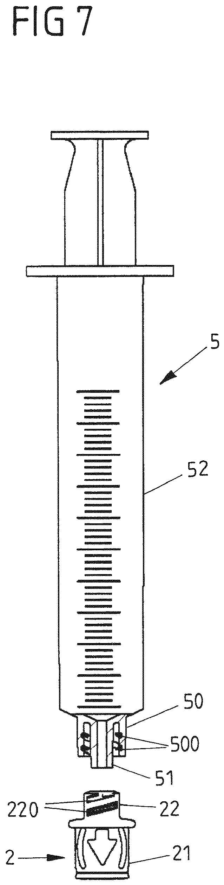

Während das an das zweite Anschlussstück 12 angesetzte Ansetzteil 3 zum Ansetzen einer einen Dorn aufweisenden Fördereinrichtung ausgestaltet ist, um durch Durchstechen eines Dichtelements mittels des Dorns einen Zugang zu dem Behältnis 1 zu schaffen und Flüssigkeit aus dem Behältnis 1 heraus zu fördern, stellen das erste Anschlussstück 11 und das daran angesetzte Ansetzteil 2 einen sogenannten nadelfreien Zugang zur Verfügung, über den ein Zuführen von Flüssigkeit in das Behältnis 1 hinein oder Flüssigkeit aus dem Behältnis heraus unter Verwendung einer Fördereinrichtung 5 (siehe Fig. 7) ohne eine Nadel vorgenommen werden kann.

Das in Fig. 1 dargestellte Behältnis besteht - in an sich bekannter Weise - aus flexiblen Folien, die entlang von Schweißnähten 100, 101 miteinander verschweißt sind. Die Anschlussstücke 11, 12 weisen jeweils insbesondere eine Schiffchenform (sogenannter Ship Shape) auf und sind jeweils mit einem verbreiterten Abschnitt 113, 121 zwischen die Folien des Behältnisses 1 eingesetzt und über eine obere Schweißnaht 101 flüssigkeitsdicht mit den Folien verschweißt. Über die Anschlussstücke 11, 12 werden somit zwei Zugänge zu dem Behältnis 1 geschaffen, über die Flüssigkeit in das Behältnis 1 eingefüllt oder aus dem Behältnis 1 entnommen werden kann.

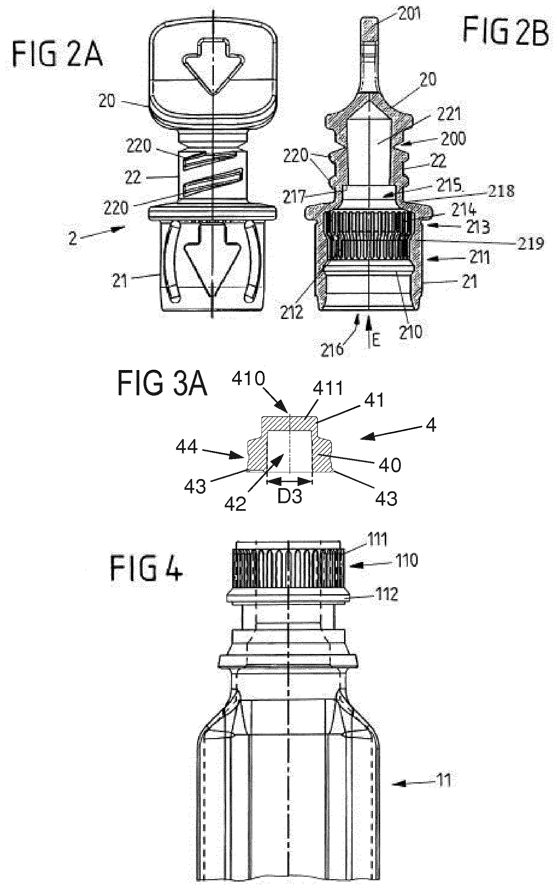

Ein Ausführungsbeispiel eines Konnektors mit einem Anschlussstück 11, einem Ansetzteil 2 und einem Dichtelement 4, das bei an das Anschlussstück 11 angesetztem Ansetzteil 2 zwischen dem Ansetzteil 2 und dem Anschlussstück 11 zu liegen kommt, zeigen Fig. 2A, 2B bis 6.

Das Ansetzteil 2 umfasst einen in seiner Grundform zylindrischen Verbindungsabschnitt 21, an den ein in seiner Grundform ebenfalls zylindrischer Gewindeabschnitt 22 anschließt. Mit dem Gewindeabschnitt 22, der an seiner äußeren zylindrischen Mantelfläche Gewindegänge 220 trägt, ist integral das Abbrechstück 20 verbunden, wobei zwischen dem Abbrechstück 20 und dem Gewindeabschnitt 22 eine Sollbruchstelle 200 ausgebildet ist, die ein definiertes Abtrennen des Abbrechstücks 20 von dem Gewindeabschnitt 22 entlang der Sollbruchstelle 200 ermöglicht.

Das Ansetzteil 2 ist integral, beispielsweise aus Kunststoff mittels Kunststoffspritzgießen, geformt.

In dem in seiner Grundform zylindrischen Verbindungsabschnitt 21 ist eine innere Öffnung 216 ausgebildet, an die eine konische, den Gewindeabschnitt 22 durchdringende Öffnung 221 anschließt. Der Durchmesser der Öffnung 221 verjüngt sich in Richtung der inneren Öffnung 216.

Das Ansetzteil 2 kann an einen Kopf 110 des Anschlussstücks 11 angesetzt werden. Dies erfolgt dadurch, dass das Anschlussstück 11 mit seinem Kopf 110 in eine Einsetzrichtung E in die Öffnung 216 eingeführt wird, bis ein Formschlusselement in Form eines umlaufenden Ringvorsprungs 112 an dem Kopf 110 formschlüssig in Eingriff mit einem Rasteingriff 210 in Form einer rillenförmigen Vertiefung innerhalb der Öffnung 216 gelangt.

An die rillenförmige Vertiefung 210 schließt innerhalb der Öffnung 216 in Einsetzrichtung E ein erster Aufnahmeabschnitt 211 an. An diesen ersten Aufnahmeabschnitt 211 wiederum schließt ein zweiter Aufnahmeabschnitt 213 an, der in Richtung des Gewindeabschnitts 22 durch einen dritten Aufnahmeabschnitt 215 fortgesetzt wird. In einem Zustand, in dem das Ansetzteil 2 an den Kopf 110 des Anschlussstücks 11 angesetzt ist, steht das Formschlusselement 112 in Form des umlaufenden Ringvorsprungs formschlüssig in Eingriff mit dem Rasteingriff 210 in Form der rillenförmigen Vertiefung innerhalb der Öffnung 216. In diesem Zustand steht der Kopf 110 über eine Verzahnung 111 in Form einer Außenzahnung zudem in Eingriff mit einer Verzahnung 212 in Form einer Innenverzahnung an dem ersten Aufnahmeabschnitt 211. Das Ansetzteil 2 ist somit gegenüber dem Anschlussstück 11 sowohl axial entlang der Einsetzrichtung E als auch drehfest um die Einsetzrichtung E festgelegt, so dass das Ansetzteil 2 in orts- und lagefest an dem Anschlussstück 11 gehalten ist.

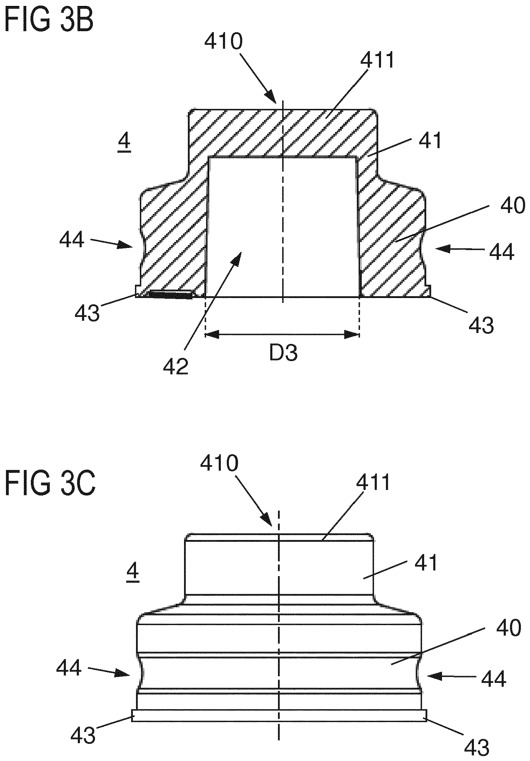

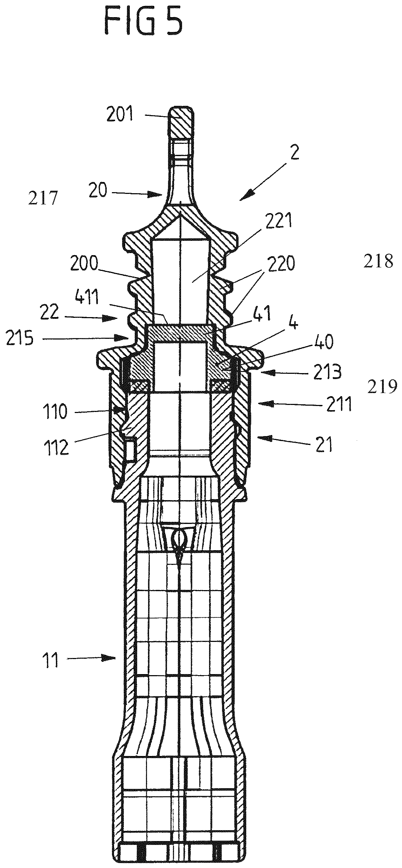

In diesem angesetzten Zustand kommt das Dichtelement 4 zwischen dem Ansetzteil 2 und dem Anschlussstück 11 zu liegen, wie dies in Fig. 5 dargestellt ist. Das Dichtelement 4 weist eine rotationssymmetrische oder eine im Wesentlichen rotationssymmetrische Form auf. Es wird gebildet durch einen im Wesentlichen zylindrischen Körper 40, an den in Einsetzrichtung E ein Dichtkopf 41 anschließt. In dem Körper 40 ist eine zylindrische Bohrung 42 mit konstantem Durchmesser D3 oder sich hin zum Dichtkopf 41 leicht verjüngender Form ausgebildet, die in Einsetzrichtung E durch den Dichtkopf 41 in dichtender Weise abgeschlossen ist (siehe z.B. Fig. 3A und 3.B).

Das Dichtelement 4 bildet eine selbstdichtende Membran aus. So ist in dem Dichtkopf 41 eine Schlitzöffnung 410 angeordnet, die den Dichtkopf 41 axial durchdringt und somit - bei Ansetzen eines Anschlussstutzens 51 einer Fördereinrichtung 5 (siehe Fig. 7) - das Dichtelement 4 öffnet, bei nicht angesetzter Fördereinrichtung 5 jedoch verschlossen ist und somit einen flüssigkeitsdichten Übergang zwischen dem Ansetzteil 2 und dem Anschlussstück 11 bereitstellt. Die Oberseite des Dichtelements 4 befindet sich im Inneren der Öffnung 221. Sie liegt unterhalb der Sollbruchstelle 200 und ist somit nicht bündig mit der Oberseite der Öffnung 221.

In aneinander angesetztem Zustand kommt das Dichtelement 4 mit seinem Körper 40 im Bereich des zweiten Aufnahmeabschnitts 213 innerhalb der Öffnung 216 des Verbindungsabschnitts 21 zu liegen, wie dies in Fig. 5 dargestellt ist. Der Körper 40 liegt dabei mit radialem Spiel in dem zweiten Aufnahmeabschnitt 213 ein, so dass bei nicht angesetzter Fördereinrichtung 5 der zylindrische Körper 40 die umfängliche Außenwandung des zweiten Aufnahmeabschnitts 213 nicht berührt.

Der zweite Aufnahmeabschnitt 213 weist einen Durchmesser D2 auf, der größer ist als der Durchmesser D1 des ersten Aufnahmeabschnitts 211. Das Dichtelement 4 liegt mit seinem Körper 40 somit mit radialem Spiel in dem zweiten Aufnahmeabschnitt 213 ein. Es wird dadurch zwischen dem ersten Aufnahmeabschnitt 211 und dem zweiten Aufnahmeabschnitt 213 eine dritte Stufe 219 gebildet. Auf diese Weise wird Raum zum Verformen des Dichtelements 4 geschaffen, so dass das Dichtelement 4 in günstiger Weise beiseite gedrängt werden kann, wenn eine Fördereinrichtung 5 an das Ansetzteil 2 angesetzt wird. Vorzugsweise besitzt der zweite Aufnahmeabschnitt 213 einen Durchmesser D2, der um 0,2 mm bis 1,5 mm größer ist als der Durchmesser D1 des ersten Aufnahmeabschnitts 211.

Der Dichtkopf 41 kommt hierbei in dem dritten Aufnahmeabschnitt 215 zu liegen und stellt auf diese Weise, durch Anlage an einer ersten Stufe 217 dieses dritten Aufnahmeabschnitts 215, einen dichtenden Übergang zu dem Ansetzteil 2 her. Zudem liegen der Dichtkopf 41 und der Dichtkörper 40 an einer zweiten Stufe 218 an.

In aneinander angesetztem Zustand kommt das Dichtelement 4 klemmend zwischen dem Ansetzteil 2 und dem Anschlussstück 11 zu liegen. Der Dichtkopf 41 stellt hierbei eine flüssigkeitsdichte Trennung zwischen der konischen Öffnung 221 des Gewindeabschnitts 22 und dem Anschlussstück 11 zur Verfügung. Die konische Öffnung 221 zur Aufnahme eines konischen Anschlussstutzens 51, beispielsweise einer Fördereinrichtung 5, vorzugsweise einer Luer- oder Luer-Lock-Spritze, ist oberhalb des Dichtelements 4 angeordnet.

Wie insbesondere aus Fig. 2 ersichtlich, weisen sowohl der erste Aufnahmeabschnitt 211 als auch der zweite Aufnahmeabschnitt 213 eine Verzahnung 212, 214 in Form einer umlaufenden Innenverzahnung auf. Während die Verzahnung 212 des ersten Aufnahmeabschnitts 211 der drehfesten Festlegung an dem Kopf 110 des Anschlussstücks 11 dient, hat die Verzahnung 214 des zweiten Aufnahmeabschnitts 213 im späteren Einsatz keine unmittelbare Funktion. Die Verzahnung 214 des zweiten Aufnahmeabschnitts 213 dient der verbesserten Entformung des Ansetzteils 2 nach dem Kunststoffspritzgießen. Bei diesem Entformen wird das Ansetzteil 2 nach dem Spritzgießen von einem Formungswerkzeug abgezogen. Um hierbei zu verhindern, dass beim Abziehen ein in das Ansetzteil 2 hineinragender, die Form der Öffnung 216 vorgebender Dorn des Formwerkzeugs die Verzahnung 212 an dem ersten Aufnahmeabschnitt 211 beschädigt (was aufgrund des vergrößerten Durchmessers des zweiten Aufnahmeabschnitts 213 nicht auszuschließen wäre), weist dieser Dorn auch im Bereich des zweiten Aufnahmeabschnitts 213 eine Verzahnung auf (die entsprechend auch an den zweiten Aufnahmeabschnitt 213 angeformt wird). Weil beim Entformen somit ein dem zweiten Aufnahmeabschnitt 213 komplementärer Verzahnungsabschnitt des Dorns aus dem Ansetzteil 2 herausgezogen wird und dieser Verzahnungsabschnitt durch die Verzahnung 212 des ersten Aufnahmeabschnitts 211 hindurch gleitet, kann eine Beschädigung der Verzahnung 212 des ersten Aufnahmeabschnitts 211 beim Entformen zuverlässig entgegengewirkt werden.

Das Dichtelement 4 weist an einer dem Körper 40 abgewandten Seite 411 des Dichtkopfs 41 eine ebene Form auf. Der Dichtkopf 41 ist somit nicht konkav geformt, wie bisher üblich, sondern an seiner dem Gewindeabschnitt 22 zugewandten Seite 411 plan, was das Ansetzen der Fördereinrichtung 5 und das Öffnen der Schlitzöffnung 410 beim Ansetzen erleichtert.

Das Dichtelement 4 weist, wie in den Fig. 3A bis 3C dargestellt, an seinem Körper 40 ein Formschlusselement 43 in Form eines umlaufenden Kranzes auf, der dazu dient, das Dichtelement 4 nach Einsetzen in die Öffnung 216 formschlüssig an dem Ansetzteil 2 zu halten, so dass das Dichtelement 4 nicht aus der Öffnung 216 herausfallen kann. Dies erleichtert das Ansetzen des Ansetzteils 2 an das zugeordnete Anschlussstück 11. Insbesondere kann das Ansetzen des Ansetzteils 2 an das Anschlussstück 11 mit an dem Ansetzteil 2 angebrachtem Dichtelement 4 erfolgen, ohne dass das Dichtelement 4 manuell in Position an dem Ansetzteil 2 gehalten werden muss. Ferner ist an der Außenseite des Dichtelements 4 eine, vorzugsweise umlaufende, Nut 44 vorhanden. Im Detail ist die Nut 44 an der Außenseite des Dichtkörpers 40 angeordnet. Dadurch kann zum Beispiel die Herstellung des Dichtelements 4 unterstützt werden. Die Abschnitte des Dichtkörpers 40, welche sich oberhalb und unterhalb der Nut 44 befinden, sind hier im Wesentlichen zylindrisch.

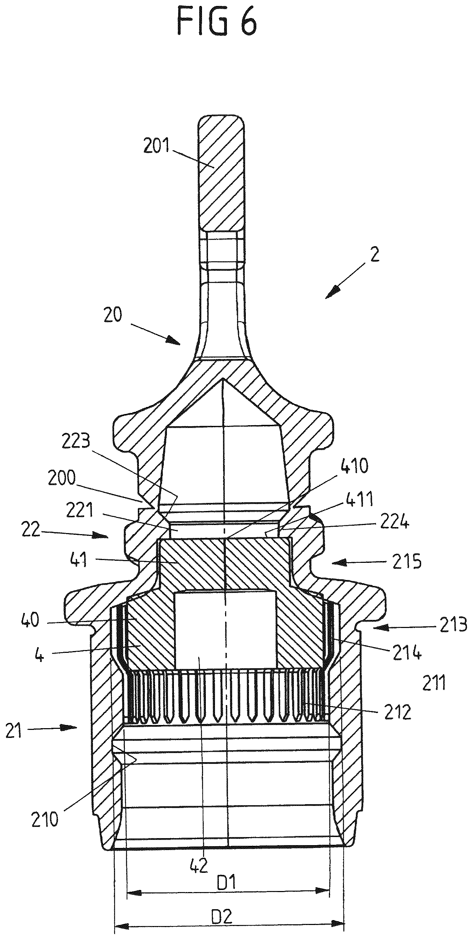

Ein anderes Ausführungsbeispiel eines Konnektors mit einem Ansetzteil 2 ist in Fig. 6 dargestellt. Bei dem Ausführungsbeispiel gemäß Fig. 6 ist innen an der Öffnung 221 des Gewindeabschnitts ein um die Öffnung 221 umlaufender, radial nach innen in die Öffnung 221 hineinragender Vorsprung 224 ausgebildet, der eine Stufe bildet, an der das Dichtelement 4 mit seiner nach außen weisenden Oberseite 411 anliegt. An der der Oberseite 411 abgewandten Seite weist der Vorsprung 224 eine Einführschräge 223 auf, die ein Einführen eines Anschlussstutzens einer Fördereinrichtung und ein Eingreifen in die Schlitzöffnung 410 des Dichtelements erleichtert. Das Ansetzteil 2 gemäß Fig. 6 ist in seiner Funktion ansonsten im Wesentlichen identisch wie das Ansetzteil 2 des Ausführungsbeispiels gemäß Fig. 2A, 2B bis 5, so dass vollumfänglich auf das vorangehend Beschriebene verwiesen werden soll.

Das Ansetzen einer Fördereinrichtung 5 an das Ansetzteil 2 erfolgt wie in Fig. 7 dargestellt. Zum Ansetzen der Fördereinrichtung 5 an das Ansetzteil 2 wird zunächst das Abbrechstück 20 an einem hierfür vorgesehenen Griffelement 201 zwischen zwei Fingern gegriffen und an seiner Sollbruchstelle 200 von dem Gewindeabschnitt 22 des Ansetzteils 2 getrennt. Nach Abbrechen des Abbrechstücks 20 ist die Öffnung 221 innerhalb des Gewindeabschnitts 22 freigegeben, so dass die Fördereinrichtung 5 mit einem in seiner äußeren Form konischen Anschlussstutzen 51 in die Öffnung 221 des Gewindeabschnitts 22, vorzugsweise dichtend, eingesetzt werden kann. Zum Verbinden und Fixieren der Fördereinrichtung 5 an dem Ansetzteil 2 wird ein Anschlusselement 50 in Form einer Überwurfmutter schraubend über Gewindegänge 500 innenseitig des Anschlusselements 50 mit den Gewindegängen 220 an dem Gewindeabschnitt 22 in Eingriff gebracht, so dass nach Aufschrauben des Anschlusselements 50 auf den Gewindeabschnitt 22 die Fördereinrichtung 5 mit dem Ansetzteil 2 verbunden ist.

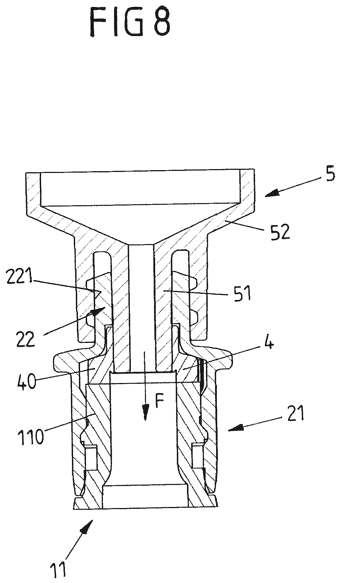

Beim Einsetzen des Anschlussstutzens 51 in die Öffnung 221 des Gewindeabschnitts 22 und beim Aufschrauben des Anschlusselements 50 in Form der Überwurfmutter auf den Gewindeabschnitt 22 gelangt der Anschlussstutzen 51 in Kontakt mit dem Dichtkopf 41 des Dichtelements 4 und durchdringt dieses, indem der Anschlussstutzen 51 die Schlitzöffnung 410 durchsticht und den Dichtkopf 41, wie dies schematisch in Fig. 8 dargestellt ist, radial beiseite drängt. Hierbei wird auch der Körper 40 verformt, indem er radial in dem zweiten Aufnahmeabschnitt 213 geweitet wird.

In an das Ansetzteil 2 angesetzter Stellung durchdringt die Fördereinrichtung 5 mit ihrem Anschlussstutzen 51 den Dichtkopf 41 des Dichtelements 4 vorzugsweise vollständig, wie dies in Fig. 8 dargestellt ist, so dass ein Durchfluss F zwischen der Fördereinrichtung 5 und dem Behältnis 1 geschaffen ist, der in seiner Öffnungsweite nicht durch das Dichtelement 4 beeinträchtigt ist.

Bei angesetzter Fördereinrichtung 5 ist der in seiner Form konisch ausgebildete Anschlussstutzen 51 in flächiger Anlage mit der entsprechend konisch geformten Öffnung 221 des Gewindeabschnitts 22, so dass hierüber der Übergang zwischen der Fördereinrichtung 5 und dem Ansetzteil 2 abgedichtet ist.

Das Dichtelement 4 kann aus einem thermoplastischen Elastomer hergestellt sein und vorzugsweise eine Shore-Härte zwischen 25 und 70, beispielsweise zwischen 30 und 60 aufweisen. Weil das Dichtelement 4 in dieser Ausgestaltung vergleichsweise steif ausgestaltet ist, kann die Fördereinrichtung 5 mit ihrem Anschlussstutzen 51 das Dichtelement 4 effizient radial beiseite drängen, insbesondere ohne dass das Dichtelement 4 sich axial dehnt. Die - im Vergleich zu bisher üblichen Dichtelementen - größere Härte des Dichtelements 4 kann somit das Ansetzen der Fördereinrichtung 5 an das Ansetzteil 2 erleichtern und das Schaffen eines Durchflusses F mit großem Strömungsdurchmesser verbessern. In einer alternativen Ausführungsform kann das Dichtelement 4 aus Polyisopren hergestellt sein.

Nach Hineinfördern einer medizinischen Flüssigkeit aus einem Spritzenkörper 52 der Spritze 5 in das Behältnis 1 kann die Fördereinrichtung 5 wiederum von dem Ansetzteil 2 entnommen werden, indem das Anschlusselement 50 gelöst und die Fördereinrichtung 5 von dem Ansetzteil 2 entfernt wird. Hierdurch wird der Anschlussstutzen 51 außer Eingriff mit der Schlitzöffnung 410 gezogen, wodurch sich die Schlitzöffnung 410 selbsttätig wieder schließt und das Dichtelement 4 somit in seinen Ausgangszustand zurückkehrt.

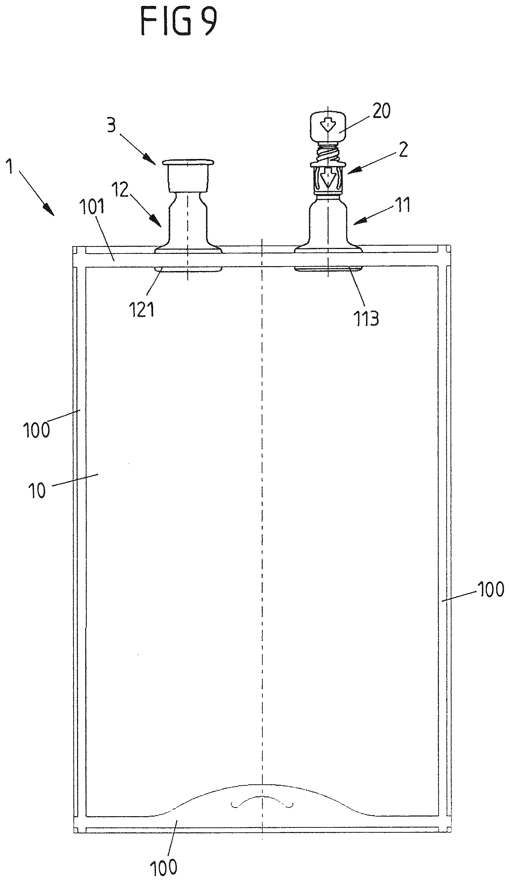

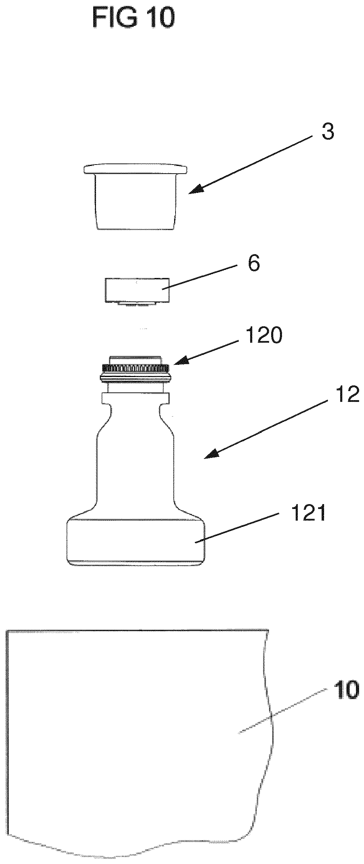

Ein weiteres Ausführungsbeispiel eines Behältnisses 1 zeigen Fig. 9 und 10.

Bei dem Ausführungsbeispiel gemäß Fig. 9 und 10 ist das Behältnis 1 wiederum als flexibler Beutel ausgestaltet, der aus flexiblen Folien gebildet ist. Die Folien sind über Schweißnähte 100, 101 miteinander verschweißt, wobei Anschlussstücke 11, 12 zweier Konnektoren mit Abschnitten 113, 121 zwischen die Folien des Beutels eingesetzt und über eine obere Schweißnaht 101 flüssigkeitsdicht mit den Folien verschweißt sind.

Über die Anschlussstücke 11, 12 werden zwei Konnektoren bereitgestellt, über die Zugänge zu dem Behältnis 1 geschaffen werden.

Bei dem dargestellten Ausführungsbeispiel ist das eine Anschlussstück 11 mit einem Ansetzteil 2 verbunden, wie es vorangehend anhand des Ausführungsbeispiels gemäß Fig. 2A, 2B bis 5 beschrieben worden ist. Bezüglich dieses Konnektors soll somit vollumfänglich auf das vorangehend Beschriebene verwiesen werden.

Das andere Anschlussstück 12 hingegen ist bei dem dargestellten Ausführungsbeispiel mit einem Ansetzteil 3 in Form einer sogenannten Blindkappe verbunden, über die das Anschlussstück 12 bei späterer Verwendung des Behältnisses 1 fest verschlossen ist, sodass ein Nutzer nicht ohne Weiteres über das Anschlussstück 12 auf das Behältnis 1 zugreifen kann.

Das Behältnis 1 des Ausführungsbeispiels gemäß Fig. 9 und 10 kann insbesondere für die Dialyse und/oder die Transfusion zum Einsatz kommen. Über das Anschlussstück 12 erfolgt bei Herstellung des Beutels eine Befüllung, wobei unmittelbar nach Befüllung die Blindkappe 3 an das Anschlussstück 12 angesetzt und das Anschlussstück 12 somit verschlossen wird. Im späteren Betrieb kann dann über das andere Anschlussstück 11 auf das Behältnis 1 zugegriffen werden, um Flüssigkeit aus dem Behältnis 1 zu entnehmen oder dem Behältnis 1 zuzuführen.

Der durch die Blindkappe 3 und das Anschlussstück 12 gebildete Konnektor ist in einer Explosionsansicht in Fig. 10 dargestellt. Das Anschlussstück 12 weist einen Kopf 120 auf, an den zum Verschließen des Anschlussstücks 12 die Blindkappe 3 angesetzt werden kann. Zwischen der Blindkappe 3 und dem Kopf 120 des Anschlussstücks 12 kommt hierbei ein Dichtelement 6 zum Einsatz, das einen flüssigkeitsdichten Verschluss des Anschlussstücks 12 gewährleistet und im Inneren der Blindkappe 3 aufgenommen ist. Das Dichtelement 6 wird bei angesetzter Blindkappe 3 klemmend zwischen der Blindkappe 3 und dem Kopf 120 gehalten, so dass auf diese Weise das Anschlussstück 12 flüssigkeitsdicht verschlossen ist.

Die Blindkappe 3 dient dazu, die Verwendung des durch das Anschlussstück 12 bereitgestellten Konnektors in späterer Benutzung des Behältnisses 1 zu verhindern. Die Blindkappe 3 kann durch einen Nutzer nicht in einfacher Weise entfernt werden und soll bei späterer Benutzung des Behältnisses 1 auf dem Anschlussstück 12 verbleiben.

Auf das Anschlussstück 11 kann bei späterer Benutzung des Behältnisses 1 hingegen zugegriffen werden, indem das Abbrechstück 20, ganz genauso wie die vorangehend beschrieben worden ist, abgebrochen wird. An das Ansetzteil 2 kann dann eine Fördereinrichtung 5 beispielsweise in Form einer Spritze oder eine Pumpe, beispielsweise einer Transfusionspumpe, angesetzt werden, um Flüssigkeit in das Behältnis 1 einzufüllen oder aus dem Behältnis 1 zu entnehmen.

Zur Herstellung des Behältnisses 1 werden zunächst die das Behältnis 1 ausbildenden, flexiblen Folien ausgerollt, aufeinander gelegt und beispielsweise bedruckt. Sodann werden die Schweißnähte 100 seitlich und am unteren Rand des Behältnisses 1 angebracht, so dass die Folien an ihren seitlichen Rändern und unten dicht miteinander verbunden werden. Anschließend werden die Folien beschnitten, so dass sich die in Fig. 9 dargestellte Beutelform ergibt.

Im Bereich der unteren Schweißnaht 100 wird sodann eine Öffnung als Aufhängung für das Behältnis 1 angebracht.

Anschließend werden die aufeinandergelegten Folien des Behältnisses 1 an ihrem oberen Rand voneinander getrennt, und die Anschlussstücke 11, 12 werden mit ihren Abschnitten 113, 121 zwischen die Folien eingeführt. Das Anschlussstück 11 wird hierbei mit daran angesetztem Ansetzteil 2 eingesetzt. Das Anschlussstück 12 hingegen wird ohne Blindkappe 3 zwischen die Folien gesetzt.

Sind die Anschlussstücke 11, 12 zwischen die Folien eingesetzt worden, wird die obere Schweißnaht 101 angebracht, um auf diese Weise die Folien miteinander und mit den Anschlussstücken 11, 12 zu verschweißen. Das Beutelinnere wird auf diese Weise flüssigkeitsdicht verschlossen. Zugänge werden über die Anschlussstücke 11, 12 bereitgestellt.

Anschließend erfolgt das Befüllen des Behältnisses 1 über das Anschlussstück 12 beispielsweise mit einer Kochsalzlösung oder einer anderen medizinischen Flüssigkeit. Ist das Befüllen beendet, wird die Blindkappe 3 mit dem Dichtelement 6 an das Anschlussstück 12 angesetzt, um auf diese Weise das Anschlussstück 12 zu verschließen. Weil auch das Ansetzteil 2 an das andere Anschlussstück 11 angesetzt ist, ist das Behältnis 1 somit flüssigkeitsdicht verschlossen.

Um das Behältnis 1 zur weiteren Verwendung auszuliefern, wird sodann das Behältnis 1 in eine zusätzliche Umhüllung eingeschweißt, indem das Behältnis 1 zwischen Folien gelegt wird und diese miteinander verschweißt werden.

Anschließend kann das Behältnis 1 in eingeschweißter Form beispielsweise sterilisiert und beispielsweise in einem Karton verpackt werden.

Zur eigentlichen Verwendung beispielsweise in einem Krankenhaus wird das Behältnis 1 dann aus dem Karton entnommen und die Folienumhüllung durch Abziehen der Folien entfernt. Über den einen Luer-Lock-Port bereitstellenden Konnektor des Anschlussstücks 11 kann auf das Behältnis 1 zugegriffen werden, um beispielsweise Flüssigkeit aus dem Behältnis 1 zu entnehmen, um das Transfusionsset zu spülen und/oder um einem Patienten zu infundieren.

Der der Erfindung zugrunde liegende Gedanke ist nicht auf die vorangehend geschilderten Ausführungsbeispiele beschränkt, sondern lässt sich grundsätzlich auch bei gänzlich anders gearteten Ausführungsformen verwirklichen.

Insbesondere kann die vorliegende Erfindung auch bei anderen Behältnissen in Form von Ampullen oder Flaschen oder dergleichen eingesetzt werden.

Grundsätzlich kann ein solches Behältnis ein oder mehrere Anschlussstücke mit ein oder mehreren daran anzuordnenden, gleichartigen oder verschiedenen Ansetzteilen aufweisen.

### Bezugszeichenliste

- 1: Beutel
- 10: Beutelkörper
- 100, 101: Schweißnaht
- 11, 12: Anschlussstück
- 110: Kopf
- 111: Verzahnung
- 112: Formschlusselement (Ringvorsprung)
- 113: Abschnitt
- 120: Abschnitt
- 2: Ansetzteil
- 20: Abbrechstück
- 200: Sollbruchstelle
- 201: Griffelement
- 21: Verbindungsabschnitt
- 210: Rasteingriff
- 211: Aufnahmeabschnitt
- 212: Verzahnung
- 213: Aufnahmeabschnitt
- 214: Verzahnung
- 215: Aufnahmeabschnitt
- 216: Öffnung
- 217: Erste Stufe
- 218: Zweite Stufe
- 218: Dritte Stufe
- 22: Gewindeabschnitt
- 220: Gewindegänge
- 221: Öffnung
- 223: Einführschräge
- 224: Vorsprung
- 3: Ansetzteil
- 30: Abbrechstück
- 4: Dichtelement
- 40: Körper
- 41: Dichtkopf
- 410: Schlitzöffnung
- 411: Seite
- 42: Innenraum
- 43: Kranz
- 44: Nut oder Vertiefung
- 5: Fördereinrichtung (Spritze)
- 50: Anschlusselement
- 500: Gewindegänge
- 51: Anschlussstutzen (Spritzenkonus)
- 52: Spritzenkörper
- 6: Dichtelement
- D1, D2, D3: Durchmesser
- E: Einsetzrichtung
- F: Fließrichtung

## Patentansprüche

1. Behältnis (1) für eine medizinische Flüssigkeit, mit
- einem Anschlussstück (11), über das eine medizinische Flüssigkeit in das Behältnis (1) hinein oder aus dem Behältnis (1) heraus förderbar ist,
- einem an das Anschlussstück (11) ansetzbaren Ansetzteil (2), das mit einer Förderreinrichtung (5) zum Fördern einer medizinischen Flüssigkeit in das Behältnis (1) hinein oder aus dem Behältnis (1) heraus verbindbar ist, und
- einem Dichtelement (4) zum Abdichten eines Übergangs zwischen dem Anschlussstück (11, 12) und dem Ansetzteil (2), wobei das Dichtelement (4) eine Schlitzöffnung (410) aufweist, die bei nicht an das Ansetzteil (2) angesetzter Fördereinrichtung (5) gegen einen Flüssigkeitsdurchtritt geschlossen und durch Ansetzen der Fördereinrichtung (5) an das Ansetzteil (2) zu öffnen ist derart, dass eine medizinische Flüssigkeit durch die Schlitzöffnung (410) hindurch förderbar ist, wobei
- das Ansetzteil (2) einen Verbindungsabschnitt (21) aufweist mit einer Öffnung (216), in die das Anschlussstück (11) zum Verbinden mit dem Ansetzteil (2) entlang einer Einsetzrichtung (E) einsetzbar ist und
- die Öffnung (216) einen ersten Aufnahmeabschnitt (211) und einen entlang der Einsetzrichtung (E) axial daran anschließenden, zweiten Aufnahmeabschnitt (213) aufweist, wobei bei an das Anschlussstück (11) angesetztem Ansetzteil (2) der erste Aufnahmeabschnitt (211) einen Kopf (110) des Anschlussstücks (11) und der zweite Aufnahmeabschnitt (213) einen Abschnitt (40) des Dichtelements (4) aufnimmt,
**dadurch gekennzeichnet, dass**
der erste Aufnahmeabschnitt (211) einen ersten Durchmesser (D1) und der zweite Aufnahmeabschnitt (213) einen zweiten Durchmesser (D2) aufweist, wobei der erste Durchmesser (D1) kleiner ist als der zweite Durchmesser (D2) und das Dichtelement (4) bei nicht an das Ansetzteil (2) angesetzter Fördereinrichtung (5) mit radialem Spiel in dem zweiten Aufnahmeabschnitt (213) aufgenommen ist.

2. Behältnis (1) nach Anspruch 1, **dadurch gekennzeichnet, dass** das Dichtelement (4) aus einem thermoplastischen Elastomer gefertigt ist, welches insbesondere eine Shore-Härte A zwischen 25 und 70, vorzugsweise zwischen 30 und 60, aufweist.

3. Behältnis (1) nach Anspruch 1, **dadurch gekennzeichnet, dass** das Dichtelement (4) aus Polyisopren gefertigt ist.

4. Behältnis (1) nach einem der vorangehenden Ansprüche, **dadurch gekennzeichnet, dass** der zweite Durchmesser (D2) des zweiten Aufnahmeabschnitts (213) um 0,2 mm bis 1,5 mm größer ist als der erste Durchmesser (D1) des ersten Aufnahmeabschnitts (211).

5. Behältnis (1) nach einem der vorangehenden Ansprüche, **dadurch gekennzeichnet, dass** der erste Aufnahmeabschnitt (211) und der zweite Aufnahmeabschnitt (213) jeweils eine sich um die Einsetzrichtung (E) herum erstreckende Innenverzahnung (212, 214) aufweisen.

6. Behältnis (1) nach einem der vorangehenden Ansprüche, **dadurch gekennzeichnet, dass** das Dichtelement (4) einen Körper (40), der bei an das Anschlussstück (11) angesetztem Ansetzteil (2) klemmend zwischen dem Anschlussstück (11) und dem Ansetzteil (2) aufgenommen ist, und einen an den Körper (40) anschließenden Dichtkopf (41), in dem die Schlitzöffnung (410) ausgebildet ist, aufweist.

7. Behältnis (1) nach vorangehendem Anspruch 6, **dadurch gekennzeichnet, dass** der Körper (40) des Dichtelements (4) einen Außendurchmesser zwischen 6 mm und 10 mm und der an den Körper (40) anschließende Dichtkopf (41) einen Außendurchmesser zwischen 4 mm und 7 mm aufweist.

8. Behältnis (1) nach einem der vorangehenden Ansprüche 6 bis 8, **dadurch gekennzeichnet, dass** der Dichtkopf (41) an einer von dem Körper (40) abgewandten Seite (411) eben, konvex oder konkav ausgebildet ist.

9. Behältnis (1) nach einem der vorangehenden Ansprüche 6 bis 9, **dadurch gekennzeichnet,**
**dass** der Körper (40) des Dichtelements (4) eine, vorzugsweise zylindrische, Bohrung (42) mit einem, vorzugsweise konstanten, Durchmesser (D3) aufweist, die bei nicht an das Ansetzteil (2) angesetzter Fördereinrichtung (5) durch den Dichtkopf (41) gegen einen Flüssigkeitsdurchtritt geschlossen ist und/oder
**dass** das Dichtelement (4) ein radial von dem Körper (40) vorstehendes Formschlusselement (43) zum Halten des Dichtelements (4) an dem Ansetzteil (2) aufweist.

10. Behältnis (1) nach einem der vorangehenden Ansprüche, **dadurch gekennzeichnet, dass** das Ansetzteil (2) einen Gewindeabschnitt (22) mit mindestens einem Gewindegang (220) zum Herstellen einer Gewindeverbindung mit der Fördereinrichtung (5) und ein an den Gewindeabschnitt (22) anschließendes Abbrechstück (20) aufweist, wobei das Abbrechstück (20) in einem mit dem Gewindeabschnitt (22) verbundenen Zustand eine Öffnung (221) des Gewindeabschnitts (22) verschließt und zum Freigeben der Öffnung (221) von dem Gewindeabschnitt (22) entfernbar ist.

11. Behältnis (1) nach Anspruch 10, **dadurch gekennzeichnet, dass** der Gewindeabschnitt (22) und das Abbrechstück (20) einstückig ausgebildet sind, wobei zwischen dem Gewindeabschnitt (22) und dem Abbrechstück (20) eine Sollbruchstelle (200) für ein Abbrechen des Abbrechstücks (20) von dem Gewindeabschnitt (22) angeordnet ist.

12. Konnektor für ein Behältnis (1) für eine medizinische Flüssigkeit nach einem der vorstehenden Ansprüche 1 bis 11, mit einem Anschlussstück (11), durch das eine medizinische Flüssigkeit förderbar ist, einem an das Anschlussstück (11) ansetzbaren Ansetzteil (2), das mit einer Förderreinrichtung (5) zum Fördern einer medizinischen Flüssigkeit durch das Anschlussstück verbindbar ist, und einem Dichtelement (4) zum Abdichten eines Übergangs zwischen dem Anschlussstück (11, 12) und dem Ansetzteil (2), wobei das Dichtelement (4) eine Schlitzöffnung (410) aufweist, die bei nicht an das Ansetzteil (2) angesetzter Fördereinrichtung (5) gegen einen Flüssigkeitsdurchtritt geschlossen und durch Ansetzen der Fördereinrichtung (5) an das Ansetzteil (2) zu öffnen ist derart, dass eine medizinische Flüssigkeit durch die Schlitzöffnung (410) hindurch förderbar ist, wobei das Ansetzteil (2) einen Verbindungsabschnitt (21) aufweist mit einer Öffnung (216), in die das Anschlussstück (11) zum Verbinden mit dem Ansetzteil (2) entlang einer Einsetzrichtung (E) einsetzbar ist und die Öffnung (216) einen ersten Aufnahmeabschnitt (211) und einen entlang der Einsetzrichtung (E) axial daran anschließenden, zweiten Aufnahmeabschnitt (213) aufweist, wobei bei an das Anschlussstück (11) angesetztem Ansetzteil (2) der erste Aufnahmeabschnitt (211) einen Kopf (110) des Anschlussstücks (11) und der zweite Aufnahmeabschnitt (213) einen Abschnitt (40) des Dichtelements (4) aufnimmt, **dadurch gekennzeichnet, dass** der erste Aufnahmeabschnitt (211) einen ersten Durchmesser (D1) und der zweite Aufnahmeabschnitt (213) einen zweiten Durchmesser (D2) aufweist, wobei der erste Durchmesser (D1) kleiner ist als der zweite Durchmesser (D2) und das Dichtelement (4) bei nicht an das Ansetzteil (2) angesetzter Fördereinrichtung (5) mit radialem Spiel an dem zweiten Aufnahmeabschnitt (213) aufgenommen ist.

13. Anordnung mit einem Behältnis (1) nach einem der vorangehenden Ansprüche 1 bis 11 oder mit einem Konnektor nach Anspruch 12 und einer Fördereinrichtung (5), die einen Anschlussstutzen (51) zum Ansetzen an das Ansetzteil (2) aufweist, wobei bei an das Ansetzteil (2) angesetzter Fördereinrichtung (5) der Anschlussstutzen (51) die Schlitzöffnung (410) des Dichtelements (4) durchdringt.

14. Anordnung nach vorangehendem Anspruch 13, **dadurch gekennzeichnet, dass** sich die Fördereinrichtung (5) im an das Ansetzstück (2) angesetzten Zustand durch die Schlitzöffnung (410) des Dichtelements (4) hindurch derart erstreckt, dass mindestens 80 %, eines Öffnungsquerschnitts in der Fördereinrichtung (5), durch welche der Flüssigkeitstransport erfolgt, freigegeben sind.

## Claims

1. Container (1) for a medical liquid, having
- a connection piece (11) by means of which a medical liquid is conveyable into the container (1) or out of the container (1),
- an attachment part (2) which is attachable to the connection piece (11) and is connectable to a conveying device (5) for conveying a medical liquid into the container (1) or out of the container (1), and
- a sealing element (4) for sealing a transition between the connection piece (11, 12) and the attachment part (2), wherein the sealing element (4) comprises a slot opening (410) which, with the conveying device (5) not attached to the attachment part, (2) is closed against a passage of liquid and, as a result of attaching the conveying device (5) to the attachment part (2), is to be opened in such a manner that a medical liquid is conveyable through the slot opening (410), wherein
- the attachment part (2) comprises a connecting portion (21) with an opening (216) into which the connection piece (11) is insertable along an insertion direction (E) for connection to the attachment part (2) and
- the opening (216) comprises a first receiving portion (211) and a second receiving portion (213) which connects axially thereto along the insertion direction (E), wherein, with the attachment part (2) attached to the connection piece (11), the first receiving portion (211) receives a head (110) of the connection piece (11) and the second receiving portion (213) receives a portion (40) of the sealing element (4),
**characterized in that**
the first receiving portion (211) comprises a first diameter (D1) and the second receiving portion (213) comprises a second diameter (D2), wherein the first diameter (D1) is smaller than the second diameter (D2) and the sealing element (4), with the conveying device (5) not attached to the attachment part (2), is received in the second receiving portion (213) with radial play.

2. Container (1) according to Claim 1, **characterized in that** the sealing element (4) is produced from a thermoplastic elastomer which comprises, in particular, a Shore Hardness A of between 25 and 70, preferably of between 30 and 60.

3. Container (1) according to Claim 1, **characterized in that** the sealing element (4) is produced from polyisoprene.

4. Container (1) according to one of the preceding claims, **characterized in that** the second diameter (D2) of the second receiving portion (213) is greater than the first diameter (D1) of the first receiving portion (211) by between 0.2 mm and 1.5 mm.

5. Container (1) according to one of the preceding claims, **characterized in that** the first receiving portion (211) and the second receiving portion (213) each comprise an inner toothing (212, 214) which extends around the insertion direction (E).

6. Container (1) according to one of the preceding claims, **characterized in that** the sealing element (4) comprises a body (40), which, with the attachment part (2) attached to the connection piece (11), is received in a clamping manner between the connection piece (11) and the attachment part (2), and a sealing head (41) which connects to the body (40) and in which the slot opening (410) is realized.

7. Container (1) according to preceding Claim 6, **characterized in that** the body (40) of the sealing element (4) comprises an outer diameter of between 6 mm and 10 mm and the sealing head (41) which connects to the body (40) comprises an outer diameter of between 4 mm and 7 mm.

8. Container (1) according to one of preceding Claims 6 to 8, **characterized in that** the sealing head (41) is realized in a flat, convex or concave manner on a side (411) remote from the body (40).

9. Container (1) according to one of preceding Claims 6 to 9, **characterized in that** the body (40) of the sealing element (4) comprises a, preferably cylindrical, bore (42) with a, preferably constant, diameter (D3) which, with the conveying device (5) not attached to the attachment part (2), is closed by the sealing head (41) against a passage of liquid and/or
**in that** the sealing element (4) comprises a positive locking element (43) which projects radially from the body (40) for holding the sealing element (4) on the attachment part (2).

10. Container (1) according to one of the preceding claims **characterized in that** the attachment part (2) comprises a threaded portion (22) with at least one thread (220) for producing a threaded connection to the conveying device (5) and a break-off piece (20) which is connected to the threaded potion (22), wherein the break-off piece (20) closes an opening (221) of the threaded portion (22) in a state connected to the threaded portion (22) and is removable from the threaded portion (22) to release the opening (221).

11. Container (1) according to Claim 10, **characterized in that** the threaded portion (22) and the break-off piece (20) are realized in one piece, wherein a predetermined breaking point (200) for breaking-off the break-off piece (20) from the threaded portion (22) is arranged between the threaded portion (22) and the break-off piece (20).

12. Connector for a container (1) for a medical liquid according to one of preceding Claims 1 to 11, having a connection piece (11), through which a medical liquid is conveyable, an attachment part (2) which is attachable to the connection piece (11) and is connectable to a conveying device (5) for conveying a medical liquid through the connection piece, and a sealing element (4) for sealing a transition between the connection piece (11, 12) and the attachment part (2), wherein the sealing element (4) comprises a slot opening (410) which, with the conveying device (5) not attached to the attachment part (2), is closed against a passage of liquid and, as a result of attaching the conveying device (5) to the attachment part (2), is to be opened in such a manner that a medical liquid is conveyable through the slot opening (410), wherein the attachment part (2) comprises a connecting portion (21) with an opening (216) into which the connection piece (11) is insertable along an insertion direction (E) for connection to the attachment part (2) and the opening (216) comprises a first receiving portion (211) and a second receiving portion (213) which connects axially thereto along the insertion direction (E), wherein, with the attachment part (2) attached to the connection piece (11), the first receiving portion (211) receives a head (110) of the connection piece (11) and the second receiving portion (213) receives a portion (40) of the sealing element (4) **characterized in that** the first receiving portion (211) comprises a first diameter (D1) and the second receiving portion (213) comprises a second diameter (D2), wherein the first diameter (D1) is smaller than the second diameter (D2) and the sealing element (4), with the conveying device (5) not attached to the attachment part (2), is received in the second receiving portion (213) with radial play.

13. Arrangement having a container (1) according to one of preceding Claims 1 to 11 or having a connector according to Claim 12, and a conveying device (5) which comprises a connecting piece (51) for the attachment to the attachment piece (2), wherein, with the conveying device (5) attached to the attachment part (2), the connecting piece (51) penetrates the slot opening (410) of the sealing element (4).

14. Arrangement according to preceding Claim 13, **characterized in that** the conveying device (5), in the state attached to the attachment part (2), extends through the slot opening (410) of the sealing element (4) in such a manner that at least 80 %, of an opening cross section in the conveying device (5), through which the liquid is transported, is exposed.

## Revendications

1. Récipient (1) pour un liquide médical, comprenant
- un raccord (11) par le biais duquel un liquide médical peut être introduit dans le récipient (1) ou ressorti du récipient (1),
- une pièce rapportée (2) pouvant être appliquée contre le raccord (11), laquelle peut être connectée à un dispositif de transport (5) pour introduire un liquide médical dans le récipient (1) ou pour le ressortir du récipient (1), et
- un élément d'étanchéité (4) pour réaliser l'étanchéité d'une transition entre le raccord (11, 12) et la pièce rapportée (2), l'élément d'étanchéité (4) présentant une ouverture en forme de fente (410) qui, lorsque le dispositif de transport (5) n'est pas appliqué contre la pièce rapportée (2), est fermée pour empêcher le passage de liquide, et doit être ouverte par l'application du dispositif de transport (5) contre la pièce rapportée (2), de telle sorte qu'un liquide médical puisse être transporté à travers l'ouverture en forme de fente (410),
- la pièce rapportée (2) présentant une portion de connexion (21) avec une ouverture (216) dans laquelle le raccord (11) peut être introduit le long d'une direction d'insertion (E) pour la connexion à la pièce rapportée (2) et
- l'ouverture (216) présentant une première portion de réception (211) et une deuxième portion de réception (213) se raccordant axialement à celle-ci le long de la direction d'insertion (E), la première portion de réception (211), lorsque la pièce rapportée (2) est appliquée contre le raccord (11), recevant une tête (110) du raccord (11), et la deuxième portion de réception (213) recevant une portion (40) de l'élément d'étanchéité (4),
**caractérisé en ce que**
la première portion de réception (211) présente un premier diamètre (D1) et la deuxième portion de réception (213) présente un deuxième diamètre (D2), le premier diamètre (D1) étant inférieur au deuxième diamètre (D2) et l'élément d'étanchéité (4), lorsque le dispositif de transport (5) n'est pas appliqué contre la pièce rapportée (2), étant reçu avec un jeu radial dans la deuxième portion de réception (213).

2. Récipient (1) selon la revendication 1, **caractérisé en ce que** l'élément d'étanchéité (4) est fabriqué en un élastomère thermoplastique qui présente notamment une dureté Shore A comprise entre 25 et 70, de préférence entre 30 et 60.

3. Récipient (1) selon la revendication 1, **caractérisé en ce que** l'élément d'étanchéité (4) est fabriqué en polyisoprène.

4. Récipient (1) selon l'une quelconque des revendications précédentes, **caractérisé en ce que** le deuxième diamètre (D2) de la deuxième portion de réception (213) est supérieur de 0,2 mm à 1,5 mm au premier diamètre (D1) de la première portion de réception (211).

5. Récipient (1) selon l'une quelconque des revendications précédentes, **caractérisé en ce que** la première portion de réception (211) et la deuxième portion de réception (213) présentent chacune une denture intérieure (212, 214) s'étendant autour de la direction d'insertion (E).

6. Récipient (1) selon l'une quelconque des revendications précédentes, **caractérisé en ce que** l'élément d'étanchéité (4) présente un corps (40) qui, lorsque la pièce rapportée (2) est appliquée contre le raccord (11), est reçu par serrage entre le raccord (11) et la pièce rapportée (2), et une tête d'étanchéité (41) se raccordant au corps (40), dans laquelle est réalisée l'ouverture en forme de fente (410).

7. Récipient (1) selon la revendication précédente 6, **caractérisé en ce que** le corps (40) de l'élément d'étanchéité (4) présente un diamètre extérieur compris entre 6 mm et 10 mm et la tête d'étanchéité (41) se raccordant au corps (40) présente un diamètre extérieur compris entre 4 mm et 7 mm.

8. Récipient (1) selon l'une quelconque des revendications précédentes 6 à 8, **caractérisé en ce que** la tête d'étanchéité (41) est réalisée sous forme plane, convexe ou concave au niveau d'un côté (411) opposé au corps (40).

9. Récipient (1) selon l'une quelconque des revendications précédentes 6 à 9, **caractérisé en ce que**
le corps (40) de l'élément d'étanchéité (4) présente un alésage (42) de préférence cylindrique, ayant un diamètre (D3) de préférence constant, qui, lorsque le dispositif de transport (5) n'est pas appliqué contre la pièce rapportée (2), est fermé par la tête d'étanchéité (41) pour empêcher le passage de liquide et/ou l'élément d'étanchéité (4) présente un élément d'engagement par correspondance de formes (43) faisant saillie radialement depuis le corps (40) pour retenir l'élément d'étanchéité (4) contre la pièce rapportée (2).

10. Récipient (1) selon l'une quelconque des revendications précédentes, **caractérisé en ce que** la pièce rapportée (2) présente une portion filetée (22) avec au moins un filet (220) pour établir une liaison filetée avec le dispositif de transport (5) et une pièce frangible (20) se raccordant à la portion filetée (22), la pièce frangible (20), dans un état connecté à la portion filetée (22), fermant une ouverture (221) de la portion filetée (22), et pouvant être enlevée de la portion filetée (22) pour libérer l'ouverture (221).

11. Récipient (1) selon la revendication 10, **caractérisé en ce que** la portion filetée (22) et la pièce frangible (20) sont réalisées d'une seule pièce, une zone destinée à la rupture (200) étant disposée entre la portion filetée (22) et la pièce frangible (20) pour séparer la pièce frangible (20) de la portion filetée (22).

12. Connecteur pour un récipient (1) pour un liquide médical selon l'une quelconque des revendications précédentes 1 à 11, comprenant un raccord (11) par le biais duquel un liquide médical peut être transporté, une pièce rapportée (2) pouvant être appliquée contre le raccord (11), laquelle peut être connectée à un dispositif de transport (5) pour transporter un liquide médical à travers le raccord, et un élément d'étanchéité (4) pour réaliser l'étanchéité d'une transition entre le raccord (11, 12) et la pièce rapportée (2), l'élément d'étanchéité (4) présentant une ouverture en forme de fente (410), qui, lorsque le dispositif de transport (5) n'est pas appliqué contre la pièce rapportée (2), est fermée pour empêcher le passage de liquide, et doit être ouverte par application du dispositif de transport (5) contre la pièce rapportée (2), de telle sorte qu'un liquide médical puisse être transporté à travers l'ouverture en forme de fente (410), la pièce rapportée (2) présentant une portion de connexion (21) avec une ouverture (216) dans laquelle le raccord (11) peut être introduit le long d'une direction d'insertion (E) pour la connexion à la pièce rapportée (2) et l'ouverture (216) présentant une première portion de réception (211) et une deuxième portion de réception (213) se raccordant axialement à celle-ci le long de la direction d'insertion (E), la première portion de réception (211), lorsque la pièce rapportée (2) est appliquée contre le raccord (11), recevant une tête (110) du raccord (11), et la deuxième portion de réception (213) recevant une portion (40) de l'élément d'étanchéité (4), **caractérisé en ce que** la première portion de réception (211) présente un premier diamètre (D1) et la deuxième portion de réception (213) présente un deuxième diamètre (D2), le premier diamètre (D1) étant inférieur au deuxième diamètre (D2) et l'élément d'étanchéité (4), lorsque le dispositif de transport (5) n'est pas appliqué contre la pièce rapportée (2), étant reçu avec un jeu radial sur la deuxième portion de réception (213).

13. Agencement comprenant un récipient (1) selon l'une quelconque des revendications précédentes 1 à 11 ou comprenant un connecteur selon la revendication 12 et un dispositif de transport (5) qui présente une tubulure de raccordement (51) pour l'application contre la pièce rapportée (2), la tubulure de raccordement (51) traversant l'ouverture en forme de fente (410) de l'élément d'étanchéité (4) lorsque le dispositif de transport (5) est appliqué contre la pièce rapportée (2).

14. Agencement selon la revendication précédente 13, **caractérisé en ce que** le dispositif de transport (5), dans l'état appliqué contre la pièce rapportée (2), s'étend à travers l'ouverture en forme de fente (410) de l'élément d'étanchéité (4) de telle sorte qu'au moins 80 % d'une section transversale d'ouverture dans le dispositif de transport (5) par le biais duquel s'effectue le transport de liquide, soient ouverts.
